(19) Europäisches Patentamt
European Patent Office
Office européen des brevets

(11) **EP 4 270 398 A1**

(12) **EUROPEAN PATENT APPLICATION**

(43) Date of publication:
**01.11.2023 Bulletin 2023/44**

(21) Application number: **23169340.9**

(22) Date of filing: **22.04.2023**

(51) International Patent Classification (IPC):
***G16B 45/00*** *(2019.01)*

(52) Cooperative Patent Classification (CPC):
**G16B 45/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.04.2022 EP 22461540**

(71) Applicant: **Instytut Chemii Fizycznej Polskiej Akademii Nauk**
**01-224 Warszawa (PL)**

(72) Inventors:
• **Rutkowski, Piotr**
**00-189 Warszawa (PL)**
• **Tabaka, Marcin**
**42-500 Bedzin (PL)**

(74) Representative: **AOMB Polska Sp. z.o.o.**
**ul. Rondo Ignacego Daszynskiego 1**
**00-843 Warsaw (PL)**

(54) **METHOD FOR VISUALIZATION OF DEVELOPMENTAL LANDSCAPES FROM SINGLE-CELL MULTIMODAL DATA**

(57) The invention is related to a method of visualization of developmental landscapes from single-cell multimodal data, comprising the following steps: a) Providing input data for two or more modalities, relating to cells being subject to investigations, each modality characterized by a set of investigated features, the data for each modality being in a form of a matrix presenting quantitative intensity of each of the investigated features in each of the cells being subject to investigations; b) Pre-processing said input data acquired in step a) for each of said two or more modalities so as to reduce the noise level of said input data; c1) Processing the pre-processed input data obtained in step b) by modelling separately each of said two or more modalities as a diffusion process with an adaptive Gaussian kernel, so as to obtain an unimodal Markov chain matrix for each of said two or more modalities; c2) Calculating multimodal weights for each of the cells being subject to investigations and for each of said two or more modalities; d) Calculating a multimodal Markov chain matrix, wherein consecutive values in each particular row of values in the multimodal Markov chain matrix, corresponding to a particular cell being subject to investigations, are calculated by adding corresponding values from the corresponding row of each of the unimodal Markov chain matrix obtained for each of said two or more modalities in step c1) weighted with the corresponding multimodal weights obtained for said cell and the modality in question in step c2); e) Eigendecomposing the multimodal Markov chain matrix obtained in step d) so as to obtain a diffusion-based dimensionally reduced Multimodal Diffusion Maps (MDM) representation of the two or more modalities; f) Visualizing a developmental landscape based on the joint Multimodal Diffusion Maps (MDM) representation obtained in step e), reduced to two dimensions or three dimensions with inclusion of cell fate probabilities.

Fig. 1

**Description**

Technical Field

The invention belongs to the field of computational genomics and bioinformatics and is related to a method for visualization of developmental landscapes from single-cell multimodal data, coming from single-cell sequencing protocols measuring simultaneously multiple sets of genome-wide features (modalities) from the same cell.

The method is to be applied in precise analysis of developmental cell processes.

Background

The invention concerns a new diffusion-based method for visualizing multimodal single-cell data of developmental biological processes called Multimodal Diffusion Maps (MDM). Multimodal data comes from single-cell sequencing protocols measuring simultaneously multiple sets of genome-wide features (modalities) from the same cell. Modalities include for example gene expression, chromatin accessibility, histone modifications, and protein levels. The invention allows to produce a low-dimensional, compressed representation ready for genomic analysis and data visualization. Such low-dimensional representations are a crucial tool in computational genomics and bioinformatics for identifying unique cell populations, understanding developmental pathways, exploring cellular and genetic variations associated with specific diseases, predicting gene functions or understanding complex biological processes at the cellular and molecular levels.

In recent years, the rapid development of high-throughput single-cell sequencing technologies has allowed the generation of large multi-modal datasets [Junyue Cao et al. "Joint profiling of chromatin accessibility and gene expression in thousands of single cells". In: Science 361.6409 (2018), pp. 1380-1385]; [Chenxu Zhu et al. "An ultra high-throughput method for single-cell joint analysis of open chromatin and transcriptome". In: Nature structural & molecular biology 26.11 (2019), pp. 1063-1070]; [Song Chen, Blue B Lake, and Kun Zhang. "High-throughput sequencing of the transcriptome and chromatin accessibility in the same cell". In: Nature biotechnology 37.12 (2019), pp. 1452-1457]. Profiling cells at multiple molecular levels enables more accurate analyses, as some biological processes may not be active in all modalities at a given time [Sai Ma et al. "Chromatin potential identified by shared single-cell profiling of RNA and chromatin". In: Cell 183.4 (2020), pp. 1103-1116]. Designing unsupervised multi-modal algorithms that reconstruct biologically accurate structures has become a significant challenge.

So far, a few approaches to creating multimodal embeddings have been utilized.

Variational autoencoders have been a popular approach, used by Cobolt, scMVP [Boying Gong, Yun Zhou, and Elizabeth Purdom. "Cobolt: Joint analysis of multimodal single-cell sequencing data". In: bioRxiv (2021)]; [Gaoyang Li et al. "A deep generative model for multi-view profiling of single-cell RNA-seq and ATAC-seq data". In: Genome biology 23.1 (2022), pp. 1-23]. An autoencoder is a model consisting of an encoder (compressing high-dimensional data to a low-dimensional latent space) and a decoder (decoding low-dimensional data to its original format). Autoencoder learns how to generate high-quality low-dimensional representations by minimizing a loss function, which compares input and decoded data. If decoded data is similar to the input data, encoded data preserves structural information well.

However, deep learning models lack explainability, which is the strength of weight-based algorithms such as WNN or MOFA+ [Yuhan Hao et al. "Integrated analysis of multimodal single-cell data". In: Cell 184.13 (2021), pp. 3573-3587]; [Ricard Argelaguet et al. "MOFA+: a statistical framework for comprehensive integration of multi-modal single-cell data". In: Genome biology 21.1 (2020), pp. 1-17]. Weights are learned parameters that inform either which modalities are most descriptive for which cells (WNN) or how features (e.g. genes) are associated with low-dimensional space factors (MOFA+).

Another group, diffusion-based methods, is based on modeling the dataset as an eigendecomposed Markov chain. Diffusion maps were applied to unimodal single-cell data [Haghverdi Laleh et. al., Diffusion maps for high-dimensional single-cell analysis of differentiation data, Bioinformatics, Volume 31, Issue 18, September 2015, Pages 2989-2998]. It was also proposed how one may merge unimodal Markov chains into a multimodal one, but without identification of the significance of each modality [Lindenbaum Ofir et. Multi-view diffusion maps, Information Fusion, Volume 55, 2020, pp. 127-149].

From the point of view of the present invention, the important aspect is also analyzing developmental processes such as cellular differentiation. Such analysis finds transition probabilities between cells. It allows ordering of cells from stem to postmitotic states accurately. For example, Monocle finds a cellular minimum spanning tree in dimensionally reduced gene expression space [Cole Trapnell et al. "The dynamics and regulators of cell fate decisions are revealed by pseudotemporal ordering of single cells". In: Nature biotechnology 32.4 (2014), pp. 381-386]. Velocyto and scVelo model developmental RNA velocity by distinguishing between unspliced and spliced mRNAs [Gioele La Manno et al. "RNA velocity of single cells". In: Nature 560.7719 (2018), pp. 494-498]; [Volker Bergen et al. "Generalizing RNA velocity to transient cell states through dynamical modeling". In: Nature biotechnology 38.12 (2020), pp. 1408-1414]. Waddington-OT employs transport theory to find optimal transitions between cells [Geoffrey Schiebinger et al. "Optimal-transport analysis of single-cell gene expression identifies developmental trajectories in reprogramming". In: Cell 176.4 (2019), pp. 928-943].

Other lineage tracing methods like diffusion maps embed cells onto a low-dimensional manifold that constructs a differentiation pseudotime [Ronald R Coifman and Stéphane Lafon. "Diffusion maps". In: Applied and computational harmonic analysis 21.1 (2006), pp. 5-3].

Known solutions have several problems, which are referred to below in the section "Detailed description."

Problem 1: Single-cell data is highly noisy and thus requires proper preprocessing steps to deal with this phenomenon.

Problem 2: As multimodal single-cell sequencing becomes widespread, high-throughput and scalable computational methods for data visualization are needed. Existing methods are moderate in terms of execution speed if the number of cells is very high.

Problem 3: Deep learning models inherently require more data to converge parameters. This may be an issue for size-limited data. It is, therefore, challenging to balance simple model architectures for limited data and more complex architectures for extensive data. Underfitting or overfitting is a highly possible issue.

Problem 4: The disadvantage of deep learning-based models is their lack of interpretability; their parameters are challenging, if possible, to interpret by humans.

Problem 5: Single-cell modalities are usually (and should not) treated as equal for all cells. For example, chromatin accessibility data should be more informative about cells in early differentiation stages. In contrast, RNA or protein data should be more descriptive of later differentiation stages.

Problem 6: New computational methods for single-cell analysis should apply to many data formats, preprocessed and unprocessed.

Summary

The invention takes multimodal single-cell sequencing data as input and produces a low-dimensional, compressed representation that can be visualized.

The invention discloses a method of visualization of developmental landscapes from single-cell multimodal data, comprising the following steps:

a) Providing input data for two or more modalities, relating to cells being subject to investigations, each modality characterized by a set of investigated features, the data for each modality being in a form of a matrix presenting quantitative intensity of each of the investigated features in each of the cells being subject to investigations;

b) Pre-processing said input data acquired in step a) for each of said two or more modalities so as to reduce the noise level of said input data;

c1) Processing the pre-processed input data obtained in step b) by modelling separately each of said two or more modalities as a diffusion process (a Markov chain) with an adaptive Gaussian kernel, so as to obtain an unimodal Markov chain matrix for each of said two or more modalities;

c2) Calculating multimodal weights for each of the cells being subject to investigations and for each of said two or more modalities;

d) Calculating a multimodal Markov chain matrix, wherein consecutive values in each particular row of values in the multimodal Markov chain matrix, corresponding to a particular cell being subject to investigations, are calculated by adding corresponding values from the corresponding row of each of the unimodal Markov chain matrix obtained for each of said two or more modalities in step c1) weighted with the corresponding multimodal weights obtained for said cell and the modality in question in step c2);

e) Eigendecomposing the multimodal Markov chain matrix obtained in step d) so as to obtain a diffusion-based dimensionally reduced Multimodal Diffusion Maps (MDM) representation of the two or more modalities;

f) Visualizing a developmental landscape based on the joint Multimodal Diffusion Maps, MDM, representation obtained in step e), reduced to two dimensions or three dimensions with inclusion of cell fate probabilities.

Preferably, providing the input data in step a) consists in measuring said data in an experiment.

Preferably, said two or more modalities are selected from: transcriptome (gene expression levels, levels of RNA molecules), proteome (protein levels, levels of protein molecules) or epigenome (chromatin accessibility peaks, histone modification states).

Preferably, the pre-processing in step b) is conducted by topic modelling with Bayesian generative methods.

Preferably, calculating multimodal weights in step c2) is conducted by comparing distances among investigated cells' nearest neighbors from investigated two or more modalities using modality-wise empirical global distributions of distances between cells (empirical cumulative distribution functions).

Preferably, the eigendecomposition in step e) is conducted by applying ARPACK Software.

Preferably, the visualisation in step f) is conducted by reducing the MDM representation to 2 or 3 dimensions with any dimension reduction algorithm or reducing the MDM representation to 2 or 3 dimensions with a force-directed layout ForceAtlas2 algorithm, including transition probabilities between cells such as Velocyto, scVelo or Waddington Optimal Transport.

Brief Description of the Drawings

Preferred embodiments of the present invention are presented in a more detailed way with reference to the attached drawing, in which:

Fig. 1 shows the overview of the invented method pipeline;
Fig. 2 shows the application of the invented method on simulated data;
Fig. 3 compares the invented method's multimodal weights' values to those achieved using WNN method on simulated data;
Fig. 4 shows the application of the invented method on multimodal single-cell data from hair follicle together with speed and structural benchmarks against state-of-the-art methods.
Fig. 5 shows that the invented method is capable of reconstructing the cell population of Huxley's layer in the inner root sheath, which other methods were not;
Fig. 6 shows the application of the invented method on multimodal single-cell data from human bone marrow.

Detailed Description

Preferred embodiments of the invention are described in details below. The examples serve only as an illustration and do not limit the scope of the present invention.

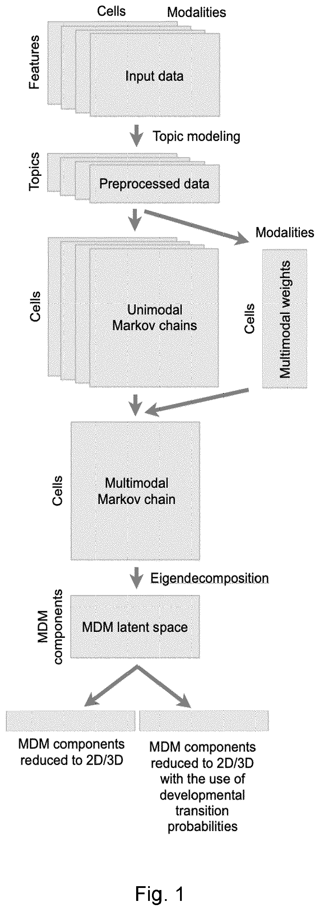

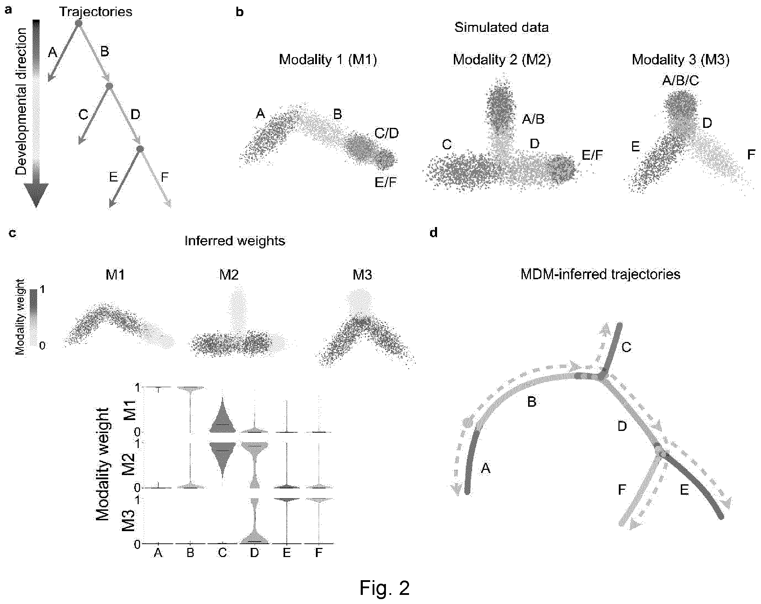

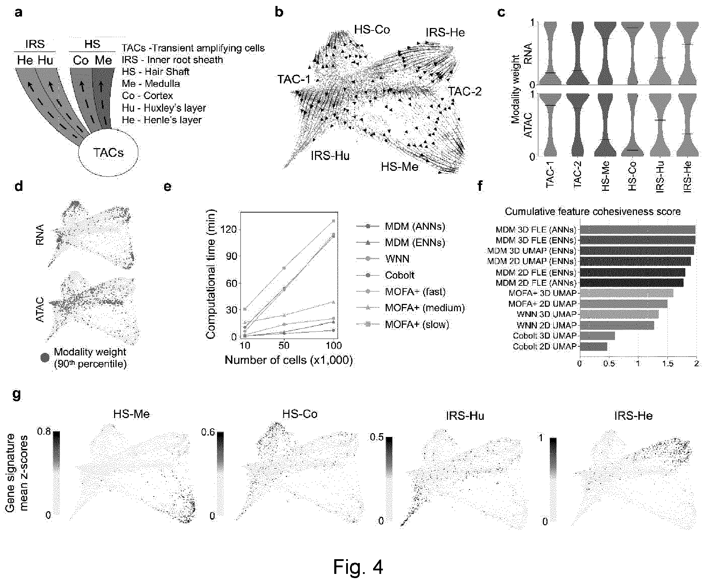

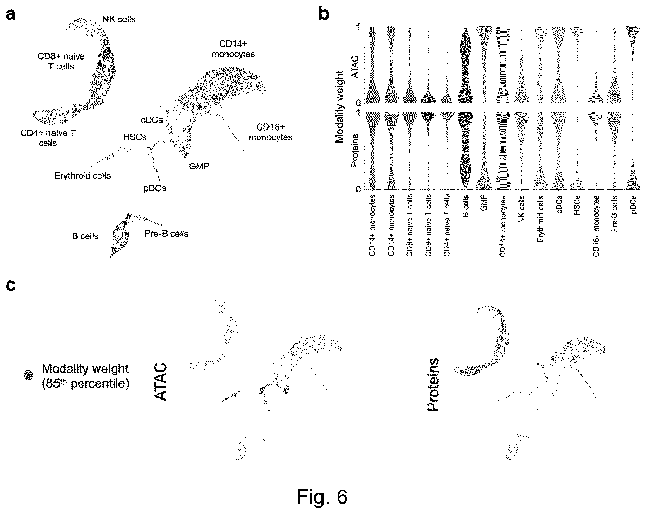

The invention, in general, concerns an analytical framework implemented with a Multimodal Diffusion Maps (MDM) method for creating joint multimodal representations of developmental multimodal single-cell data and integrating the created representations with cell-cell transition probabilities such as RNA velocyto kinetics or optimal transport (Fig. 1). The approach is based on learning cell-specific modality weights that measure the contribution of each modality to the local structure of cell neighborhoods. The performance and explainability of the algorithm is demonstrated on an artificial dataset (Fig. 2-3). The approach extends the idea from VeloViz [Lyla Atta, Arpan Sahoo, and Jean Fan. "VeloViz: RNA velocity-informed embeddings for visualizing cellular trajectories". In: Bioinformatics 38.2 (2022), pp. 391-396] and uses probabilities of transitions between cells for more accurate visualizations. The method is generalized to use any cellular transition probabilities from any method (such as Velocyto, scVelo or Waddington Optimal Transport). The performance of the invention is presented on hair follicle (Fig. 4-5) and hematopoiesis (Fig.6) data.

How the present invention solves the state-of-the-art problems

The solution to problem 1: The invented method's models are trained on modalities preprocessed with the Latent Dirichlet Allocation (LDA) algorithm [Blei David M. et al., Latent Dirichlet Allocation, Journal of Machine Learning Research 3 (2003) 993-1022]. LDA is a generative Bayesian topic modeling algorithm that compresses each modality into information-rich low-dimensional topics. Such preprocessing approach significantly removes the noise from the single-cell data and is applied to all modalities to preserve data continuity.

The solution to problem 2: In the invented method, constructed Markov chains are sparse, as cell affinities are computed only between cells' nearest neighbors, not globally. This limits the usage of memory and computational resources because of sparse data structures. The invented method is programmed for parallel computing - model training is split between multiple processors, increasing training speed on more potent machines. Training speed comparison on the bimodal hair follicle dataset between the invented method and other state-of-the-art methods using the 16CPU machine is shown in Fig. 4e.

The solution to problem 3: The invented method is diffusion-based, not deep learning-based. This enables trained models to perform well even on limited data without altering the model's architectural design. Training of a model does not rely on optimizing a loss function [Coifman Ronald R., Lafon Stéphane, Diffusion Maps, Applied and Computational Harmonic Analysis, Volume 21, Issue 1, July 2006, Pages 5-30] but on constructing a Markov chain and then eigendecomposing it to create a low-dimensional embedding.

The solution to problem 4: One of the core elements of the invented method is multimodal weights, which provide a highly-interpretable distribution of modalities' informativeness for each cell. This step dramatically enhances the invented method's explainability, which is shown in Fig. 2c, 3a, 4c, 4d, 6b and 6c.

The solution to problem 5: The invented method uses cell-specific multimodal weights, which for each cell portray how informative modalities are about cell differentiation. Then, each modality is modeled as a Markov chain. Unimodal Markov chains are weighted and combined into a multimodal Markov chain, which is then eigendecomposed. The weighing step allows for modalities to be more influential on the same cells than others.

The solution to problem 6: The invented method can take as an input any numerical data such that observations are rows and features are columns. The invented method:

(1) computes the nearest neighbors in each modality individually,
(2) computes multimodal weights based on distances between neighbors,
(3) calculates distance-based Markov affinities between neighbors.

Steps (1)-(3) can be applied to any numerical data and are not limited to specific data formats. Hence, models can be trained on raw, unprocessed count matrices or denoised preprocessed data, i.e., using LDA. The latter preprocessed data format is handy, as it vastly denoises the data. Not all algorithms accept all data formats. For example, MOFA+ may not converge when trained on compressed preprocessed data achieved with, e.g., LDA.

The solution to problem 7: The invented method is designed to portray developmental processes in single-cell data. During the computation of multimodal weights, higher values are assigned to modalities that stretch distances between the cell and its neighborhood more than the remaining modalities. Distances between two cells are compared across modalities thanks to modality-specific empirical cumulative distribution functions of distances between cells. Multimodal weights specify how informative modalities are about each cell's differentiation, which produces developmentally accurate embeddings. Additionally, components of the invented method can be visualized using developmental transition probabilities between cells. Such transitions can be computed using techniques such as Velocyto [La Manno G. et al., RNA velocity of single cells, Nature, 560, pages 494-498 (2018)], scVelo [Bergen V., Generalizing RNA velocity to transient cell states through dynamical modeling, Nature Biotechnology volume 38, pp. 1408-1414 (2020)], or Waddington Optimal Transport [Schiebinger G. et al., Optimal-Transport Analysis of Single-Cell Gene Expression Identifies Developmental Trajectories in Reprogramming, Cell, Volume 176, Issue 4, 7 February 2019, pp. 928-943]. Such visualization procedure further sorts cells locally along the cell transition probability stream.

Detailed description of the method

***Multimodal Diffusion Maps***

*Introduction*

Multimodal data $\mathcal{M}$ consists of $n_{mod}$ modalities.

$$\mathcal{M} = \{\mathbf{M}^m\}_{m=1...n_{mod}}, \mathbf{M}^m \in \mathbb{R}^{n_{cells}, n_{features\, m}} \tag{1}$$

The superscript denotes a modality to which an object relates, and the subscripts relate to coordinates - first to row, second to a column. For example, $\mathbf{M}_{i,j}^m$ represents a value from $i^{th}$ row and $j^{th}$ column of $m^{th}$ modality matrix.

$d: \mathbb{R}^k \times \mathbb{R}^k \to \mathbb{R}_{\geq 0}$ is a distance metric (the Euclidean distance is used).

*Multimodal weights*

We interpret cell differentiation as the process of cellular movement in the single-cell expression's latent space. Expanding intercellular distances indicate the presence of a developmental process, quantified by multimodal weights $\mathbf{w}^m \in \mathbb{R}^{n_{cells}}$. Weights rank cross-modality cell neighborhoods' reach respecting modalities' global structures. Higher weights indicate a higher probability of the developmental process occurrence.

Modalities are scored using empirical cumulative distribution functions (ECDF) of modalities' intercellular distances. ECDF values of distances between the cell and its cross-modality neighbors allow exploring cells locally form a global perspective. $s_c^m$, $m^{th}$ modality's score for cell $c$, is a sum of median neighborhood distance ECDF values for remaining modalities. A higher sm means that $m^{th}$ modality expands more cell $c$'s cross-modality neighborhoods.

$$s_c^m = \sum_{l \in \{1 \ldots n_{mod}\}, l \neq m} median(ECDF^m(\mathbf{d}_c^{m,l}))  \tag{2}$$

$\mathbf{d}_c^{m,l}$ is a vector of distances between cell $c$ and its $l^{th}$ modality neighbors in the latent space of $m^{th}$ modality.

We estimate $ECDF^m \colon \mathbb{R}_{\geq 0} \to [0, 1]$ by sampling uniformly P pairs of cell embeddings (P depends on total number of cells, usually P=1000 is enough to generate robust results) from $\mathbf{M}^m$ and calculating distances between them.

Scores are averaged for each cell over the highest-score modality's neighbors and normalized using a softmax function with parameter $\alpha$ (the value of $\alpha$ should be greater or equal than 1, here is used $\alpha$ =10).

$$\mathbf{w}_c^m = \frac{e^{\alpha s_c^m}}{\sum_{m=1 \ldots n_{mod}} e^{\alpha s_c^m}}  \tag{3}$$

$$\sum_{m=1 \ldots n_{mod}} \mathbf{w}_c^m = 1  \tag{4}$$

*Algorithm description*

The algorithm is a multimodal generalization of the Diffusion Maps [Ronald R Coifman and Stéphane Lafon. "Diffusion maps". In: Applied and computational harmonic analysis 21.1 (2006), pp. 5-30], where data is eigendecomposed and modelled as a Markov chain probabilistic network. The Multimodal Diffusion Maps (MDM) calculate a weighted multimodal Markov chain. The underlying idea behind the Markov chain modelling is that high-affinity cells (cells close to each other in single-cell expression latent space) can switch states. A density-adjusted symmetric Gaussian kernel $\kappa$ calculates affinity between cells and their nearest neighbors.

$$\kappa\left(\mathbf{M}_i, \varepsilon_i, \mathbf{M}_j, \varepsilon_j\right) = e^{-\frac{d(\mathbf{M}_i, \mathbf{M}_j)^2}{\varepsilon_i \varepsilon_j}}  \tag{5}$$

$\varepsilon_c$, cell c's distance to its $N^{th}$ neighbor (usually N=20 is enough for robust results), accounts for local density. $m^{th}$ modality's Markov chain is represented as a kernel matrix $\mathbf{K}^m \in \mathbb{R}^{n_{cells}, n_{cells}}$, where $\mathbf{K}_{i,j}^m$ is a weighted affinity between $j^{th}$ and $j^{th}$ cells.

$$\mathbf{K}_{i,j}^m = \mathbf{K}_{j,i}^m = \frac{\mathbf{w}_i^m + \mathbf{w}_j^m}{2} \kappa(\mathbf{M}_i^m, \varepsilon_i^m, \mathbf{M}_j^m, \varepsilon_j^m)  \tag{6}$$

The multimodal Markov chain $\mathbf{K}$ is an element-wise sum of row-normalized weighted kernel matrices. $\mathbf{D}^m$ denotes a diagonal matrix such that $\mathbf{D}_{i,i}^m = \sum_j \mathbf{K}_{i,j}^m$.

$$\mathbf{K} = \sum_{m=1 \ldots n_{mod}} \mathbf{D}^{m-1} \mathbf{K}^m  \tag{7}$$

For calculating eigenvalues, $\mathbf{K}$ is converted to a matrix $\hat{\mathbf{K}}$ that is symmetric ($\hat{\mathbf{K}} = \hat{\mathbf{K}}^T$), positive semi-definite ($\forall_{v \in \mathbb{R}^{n_{cells}} v^T \hat{\mathbf{K}} v \geq 0}$) and non-negative ($\hat{\mathbf{K}} \geq 0$). Listed properties guarantee for $\hat{\mathbf{K}}$ to be a Hermitian matrix with real and non-negative eigenvalues. $\mathbf{K}$ is row-normalized and then symmetrized along the diagonal to meet the abovementioned conditions.

$$\hat{\mathbf{K}} = \mathbf{D}^{-1}\mathbf{K} + (\mathbf{D}^{-1}\mathbf{K})^T  \tag{8}$$

$\hat{\mathbf{K}}$ is eigendecomposed to a product of $\mathbf{Q} \in \mathbb{R}^{n_{\text{cells}}, n_{\text{cells}}}$ , the eigenvector matrix ($\mathbf{Q}_{:,j}$ is the $j^{\text{th}}$ eigenvector), $\boldsymbol{\Lambda} \in \mathbb{R}^{n_{\text{cells}}}$ , the eigenvalue vector, and $\mathbf{Q}^{-1}$.

$$\hat{\mathbf{K}} = \mathbf{Q}\boldsymbol{\Lambda}\mathbf{Q}^{-1} \qquad (9)$$

Let $\hat{\Lambda}$ be the sorted eigenvalue vector with a decreasing order and $\hat{\mathbf{Q}}$ the eigenvector matrix with columns sorted accordingly. The $n_{\text{dim}}$-dimensional MDM output, $\text{MDM}(\mathcal{M}) \in \mathbb{R}^{n_{\text{cells}}, n_{\text{dim}}}$ , is defined as follows.

$$\text{MDM}(\mathcal{M}) = \hat{\boldsymbol{\Lambda}}^T_{:,2:n_{\text{dim}}+1} \hat{\mathbf{Q}}_{:,2:n_{\text{dim}}+1} \qquad (10)$$

The present implementation uses scikit-learn [F. Pedregosa et al. "Scikit-learn: Machine Learning in Python". In: Journal of Machine Learning Research 12 (2011), pp. 2825-2830], an ARPACK [Richard B Lehoucq, Danny C Sorensen, and Chao Yang. ARPACK users' guide: solution of large-scale eigenvalue problems with implicitly restarted Arnoldi methods. SIAM, 1998] wrapper, for the computation of top eigenvalues and corresponding eigenvectors. $n_{\text{dim}}$ + 11 components are computed (the quality of eigenvectors may degrade as eigenvalues get smaller; hence, 10 more than necessary eigenvectors are computed and then discarded), and $n_{\text{dim}}$ (from $2^{\text{nd}}$ to ($n_{\text{dim}}$ + 1)$^{th}$ is returned. $\hat{\Lambda}_1$, the largest eigenvalue, is non-informative in diffusion maps [Aleh Haghverdi, Florian Buettner, and Fabian J Theis. "Diffusion maps for high-dimensional single-cell analysis of differentiation data". In: Bioinformatics 31.18 (2015), pp. 2989-2998]. Also, to counter the computational quality degradation of smallest calculated eigenvectors, $\hat{\Lambda}_{n\text{dim}+2:n\text{dim}+11}$ is omitted.

*Visualizing MDM components*

MDM components can be reduced to 2D or 3D embeddings using any dimension reduction algorithm. The method (Ocelli) has built-in wrappers for UMAP [Leland McInnes, John Healy, and James Melville. "Umap: Uniform manifold approximation and projection for dimension reduction". In: arXiv preprint arXiv:1802.03426 (2018)] (recommended for data with multiple clusters) and ForceAtlas2 [Mathieu Jacomy et al. "ForceAtlas2, a continuous graph layout algorithm for handy network visualization designed for the Gephi software". In: PloS one 9.6 (2014), e98679] (recommended for well-connected data). ForceAtlas2 requires MDM components represented as a graph, and Ocelli provides two graph construction methods. 1) The nearest neighbors-based graph connecting MDM neighbors. 2) The transitions-based graph connecting a cell to its MDM neighbors with the highest developmental transition probabilities [Gioele La Manno et al. "RNA velocity of single cells". In: Nature 560.7719 (2018), pp. 494-498]; [Volker Bergen et al. "Generalizing RNA velocity to transient cell states through dynamical modelling". In: Nature biotechnology 38.12 (2020), pp. 1408-1414]; [Geoffrey Schiebinger et al. "Optimal-transport analysis of single-cell gene expression identifies developmental trajectories in reprogramming". In: Cell 176.4 (2019), pp. 928-943]. MDM components are responsible for capturing the global structure of the data while transitions order cells locally. If the number of non-zero transitions in the cell's MDM neighborhood is less than the target number of graph edges, the remaining edges link to MDM's yet unconnected nearest neighbors. Alternatively, if cell timestamps are specified, in the last step of the graph construction, MDM neighbors are selected among cells from the subsequent timestamp (exception: for cells from a terminal timestamp, neighbors are from the same timestamp).

**Preprocessing and analysis details for each dataset.**

*Hair follicle (HF) SHARE-seq dataset.*

This HF dataset is a subset of a female mouse dorsal skin multimodal SHARE-seq dataset from [Sai Ma et al. "Chromatin potential identified by shared single-cell profiling of RNA and chromatin". In: Cell 183.4 (2020), pp. 1103-1116] and contains 7160 cells.

The RNA-seq count matrix with unspliced and spliced layers was generated from BAM files using Velocyto [Gioele La Manno et al. "RNA velocity of single cells". In: Nature 560.7719 (2018), pp. 494-498]. We kept transit-amplifying cells (labeled *TAC-1*, *TAC*-2), inner root sheath (IRS) cells (labeled IRS), medulla cells (labeled *Medulla*), and hair shaft (HS) cells (labeled *Hair Shaft-cuticle. cortex*). When filtering the expression matrix, the following was performed: 1) Malat1, Gm42418, and AY036118 genes were removed, associated with RNA contamination, 2) cell doublets were removed using Scrublet [Samuel L Wolock, Romain Lopez, and Allon M Klein. "Scrublet: computational identification of cell doublets in single-cell transcriptomic data". In: Cell systems 8.4 (2019), pp. 281-291] with an estimated collision rate from [Sai Ma et al. "Chromatin potential identified by shared single-cell profiling of RNA and chromatin". In: Cell 183.4 (2020), pp. 1103-1116], 3) cells with less than 50 expressed genes were removed, 4) genes with less than 50 counts were removed, 5) genes highly correlated with the gene signature of cell proliferation from [Geoffrey Schiebinger et al. "Optimal-transport analysis of single-cell gene expression identifies developmental trajectories in reprogramming". In: Cell 176.4 (2019), pp. 928-943] were removed. In step 5), Pearson correlations between log-normalized genes' expression and the mean z-scores of the proliferation gene signature were computed. Genes with a Pearson correlation larger than 0.18 were discarded. The resulting RNA-seq count matrix had 7160 cells and 6731 genes. Next, there were computed 50 LDA topics (on the whole count matrix) and scVelo's [Volker Bergen et al. "Generalizing RNA velocity to transient cell states through dynamical modeling". In: Nature biotechnology 38.12 (2020), pp. 1408-1414] stochastic RNA velocity transitions (on 1,000 1og-normalized highly-variable genes). The ATAC-seq count matrix was generated using Signac [Tim Stuart et al. "Single-cell chromatin state analysis with Signac". In: Nature Methods (2021)] by mapping chromatin accessibility fragments to gene regions, including the 2kb upstream. Next, genes with less than 50 counts and genes correlated with the proliferation gene signature (the same genes as in RNA-seq) were filtered out. The resulting count matrix had 7160 cells and 17,495 genes. Then, 50 LDA topics on the count matrix were calculated. There were computed 20 MDM components on RNA-seq's and ATAC-seq's LDA embeddings using 20-nearest-neighbor multimodal weights. The MDM representation was reduced to 3D with ForceAtlas2 (3-edge-per-cell velocity-based graph) and then projected to 2D.

*Human bone marrow (HBM) ASAP-seq dataset.*

This HBM dataset is a multimodal ASAP-seq dataset from [Eleni P Mimitou et al. "Scalable, multimodal profiling of chromatin accessibility, gene expression and protein levels in single cells". In: Nature biotechnology 39.10 (2021), pp. 1246-1258] and contains 10,927 cells.

There were used published [Eleni P Mimitou et al. "Scalable, multimodal profiling of chromatin accessibility, gene expression and protein levels in single cells". In: Nature biotechnology 39.10 (2021), pp. 1246-1258] preprocessed count matrices: the protein count matrix with 238 protein tags and the ATAC-seq fragment counts converted to gene expression matrix with 3000 genes. 10 MDM components on proteins' and ATAC-seq's LDA embeddings (15 topics per modality) were computed using 70-nearest-neighbor multimodal weights. The MDM representation was reduced to 2D using UMAP.

### Benchmarks

*Benchmarking simulated data*

Two simulated developmental datasets were investigated.

The trimodal binary tree (BT) dataset had 6,000 observations grouped into six types (A-F). Observations were sampled from 3D Gaussian distributions and downsampled to 1,000 cells per type proportionally to local density: $P(x$ gets downsampled$) \propto (x$'s distance to its $25^{th}$ neighbor$)^3$. A developmental structure was introduced by ordering observations based on their distance to manually selected development starting points. Then, noise was added by permuting every 100 cells along the pseudotime. 10 MDM components were computed using 20-nearest-neighbor multimodal weights. The MDM representation was reduced to 2D using ForceAtlas2 (30-edge-per-cell nearest neighbor graph).

The bimodal sparse transitions (ST) dataset had 4,500 observations grouped into nine types (A-I). Observations A-C in the first modality were sampled uniformly from 3D segments, while all others originated from density-downsampled 3D Gaussian distributions (500 observations per type). Observations were ordered as in the BT dataset. 20 MDM components were computed using 60-nearest-neighbor multimodal weights. The MDM representation was reduced to 2D using ForceAtlas2 (60-edge-per-cell nearest neighbor graph).

The quality of multimodal weights was examined by comparing them to the ground truth. Observation's ground truth weights are 1 for *the best-differentiating* modality and 0 for others. *Best-differentiating* modalities are assigned to observation types as follows. For BT: modality 1 - types A and B, modality 2 - types C and D, modality 3 - types E and F. For ST: modality 1 - types A-C, modality 2 - types D-I. MDM's and Weighted Nearest Neighbors' (WNN) [Yuhan Hao et al. "Integrated analysis of multimodal single-cell data". In: Cell 184.13 (2021), pp. 3573-3587] weights were compared (trained on 60 nearest neighbors) to the ground truth using the mean squared error (MSE).

*Benchmarking scalability*

Scalability is a significant challenge for emerging computational methods in high-throughput single-cell sequencing. Three benchmark datasets were constructed, comprising 10, 50, and 100 thousand cells sampled uniformly (counts for each cell were randomly altered by adding counts or subtracting existing counts; 10 counts per cell were altered) from the HF dataset [Sai Ma et al. "Chromatin potential identified by shared single-cell profiling of RNA and chromatin". In: Cell 183.4 (2020), pp. 1103-1116]. There was investigated the scalability of WNN [Yuhan Hao et al. "Integrated analysis of multimodal single-cell data". In: Cell 184.13 (2021), pp. 3573-3587], MOFA+ [Ricard Argelaguet et al. "MOFA+: a statistical framework for comprehensive integration of multi-modal single-cell data". In: Genome biology 21.1 (2020), pp. 1-17], Cobolt [Boying Gong, Yun Zhou, and Elizabeth Purdom. "Cobolt: integrative analysis of multimodal single-cell sequencing data". In: Genome biology 22.1 (2021), pp. 1-21], and MDM by running these methods on log-normalized RNA-seq and ATAC-seq count matrices. All experiments were conducted on a 16-CPU machine. Only models' training time was measured, omitting data preprocessing or plotting (UMAP or ForceAtlas2). The training included: (WNN) construction of the multimodal graph, (MOFA+) computation of 20 factors in three convergence modes: fast, medium, and slow, (Cobolt) computation of 20 latent space dimensions, and (MDM) computation of multimodal weights and 20 MDM components using two nearest neighbors search methods: scikit-learn [F. Pedregosa et al. "Scikit-learn: Machine Learning in Python". In: Journal of Machine Learning Research 12 (2011), pp. 2825-2830] (accurate nearest neighbors) and NMSLIB [Leonid Boytsov and Bilegsaikhan Naidan. "Engineering Efficient and Effective Non-metric Space Library". In: Similarity Search and Applications - 6th International Conference, SISAP 2013, A Coruña, Spain, October 2-4, 2013, Proceedings. Ed. by Nieves R. Brisaboa, Oscar Pedreira, and Pavel Zezula. Vol. 8199. Lecture Notes in Computer Science. Springer, 2013, pp. 280-293] (approximate nearest neighbors).

*Benchmarking cohesiveness of features*

RNA-seq signatures and ATAC-seq motifs are a powerful aid in understanding the biology behind single-cell data. A benchmark is introduced, evaluating low-dimensional embeddings regarding how well they portray gene signatures and chromatin accessibility motifs (which we call features). Feature activity in a cell is measured as a mean expression z-score for RNA-seq gene signatures or a motif variability score for ATAC-seq motifs. The present benchmarking approach is based on two notions.

Firstly, close neighborhoods of highly feature-active cells should also be feature-active. 5% of most feature-active cells are selected, and the cell's score $\psi_{cell}$ is defined as a mean feature activity score among its three nearest neighbors.

Secondly, feature-active cells should be located in a cohesive embedding region; disconnected high-activity clusters may indicate a disorganized embedding. A density-based clustering algorithm DBSCAN [Martin Ester et al. "A density-based algorithm for discovering clusters in large spatial databases with noise." In: kdd. Vol. 96. 34. 1996, pp. 226-231] is employed to establish a connective structure among the 5% of most active cells. DBSCAN creates a connectivity graph $G$ with edges between cells within $\varepsilon$ distance from each other. $\varepsilon$ is set to embedding's estimated median distance between cells based on 1,000 sampled pairs of cells. The median distance is considered a fair threshold of proximity between cells.

Each connected component $G \in \mathcal{G}$ is scored using $\psi_G$. A lower $\psi_G$ indicates a better cohesiveness within $G$.

$$\Psi_G = exp(-\textstyle\sum_{cell \in G} \Psi_{cell}) \qquad (11)$$

Then, a total cohesiveness score $\Phi$ of an embedding over $\mathcal{G}$ 's all connected components is computed.

$$\Phi = -\log \left( \sum_{G \in \mathcal{G}} \Psi_G \right)$$

$\Phi$ penalizes graphs with multiple connected components. Note that the scale is inverted; higher values indicate better-connected and locally more consistent feature portrayal.

Benchmarking was carried out on the HF dataset [Sai Ma et al. "Chromatin potential identified by shared single-cell profiling of RNA and chromatin". In: Cell 183.4 (2020), pp. 1103-1116]. For ATAC-seq, 150 most variable motifs were used, computed using chromVAR [Alicia N Schep et al. "chromVAR: inferring transcription-factor-associated accessibility from single-cell epigenomic data". In: Nature methods 14.10 (2017), pp. 975-978] from the fragment count matrix. For RNA-seq, a community-finding Infomap algorithm was ran using the igraph package on inverse covariance matrix (computed using glasso package) [Friedman, Jerome, Trevor Hastie, and Robert Tibshirani. "Sparse inverse covariance estimation with the graphical lasso." Biostatistics 9.3 (2008): 432-441.] to cluster highly-dependent gene expression profiles. We kept gene clusteres with more than 10 genes, resulting in 9 gene signatures for further analysis.

Multiple multimodal analysis workflows were benchmarked utilizing WNN, MOFA+, Cobolt, and MDM. The filtered count matrices of RNA-seq (7160 cells, 6731 genes) and ATAC-seq (7160 cells, 17,495 genes) were preprocessed according to the suggestions of the workflows' authors. A multimodal graph was constructed for WNN using 50 principal components of 1,000 log-normalized highly variable genes per modality. For MOFA+, 20 factors on 1,000 highly variable genes per modality were calculated. For Cobolt, 20 latent components on count matrices with removed genes with total counts smaller than 70% quantile or higher than 99% quantile were trained. For MDM, 20 components on 50 LDA topics per modality using scikit-learn and NMSLIB were trained. Each workflow's output was then reduced to 2 or 3 dimensions using UMAP with identical parameters (20 nearest neighbors). Additionally, velocity-based ForceAtlas2 embeddings were produced for MDM using scVelo's RNA velocity transitions trained on 1,000 log-normalized highly variable genes. Cohesiveness scores over gene signatures for each evaluated embedding were summed and divided by a maximum score so that the highest-scoring embedding scored 1. Then, analogically ATAC-seq motif scores were summed and scaled. The final multimodal cohesiveness score was a sum of scaled RNA-seq and ATAC-seq scores with a maximum possible score of 2.

### Applications of the method on a simulated multimodal dataset

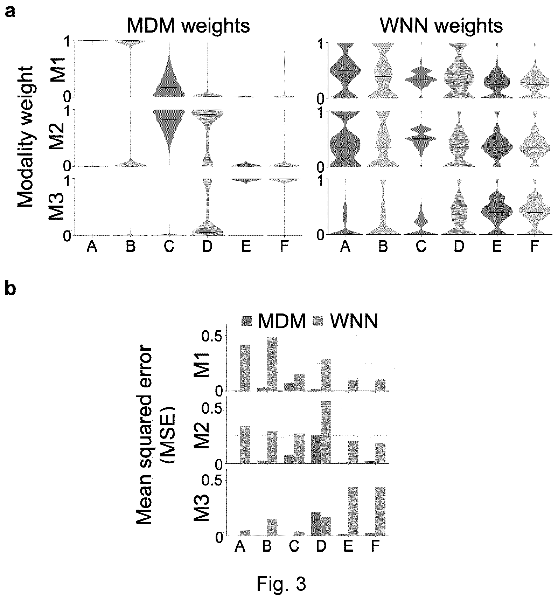

The first application of the method on a simulated multimodal dataset is shown in Fig. 2. The dataset observations are grouped into six types, A-F, forming a binary tree-like developmental process (Fig. 2a). Data is encoded in three modalities (Fig. 2b) with each observation represented as a dot in each modality. Modality 1 distinguishes well between observations of types A and B, while types C-F are mixed. Similarly, modality 2 distinguishes between types C and D, and modality 3 between types E and F. The invented method correctly learns for each observation which modality is most informative (Fig. 2c), indicated by multimodal weight distribution. Then, a joint embedding is visualized in 2 dimensions (Fig. 2d). The resulting joint multimodal embedding captures all developmental lineages A-F well separated, with clear branching points between them. The joint visualization allows us tofor capture the whole tri-modal process on a single plot, while every single modality on its own shows only two clear lineages. The performance of the invented method can be evaluated using multimodal weights (Fig. 3). Fig. 3a shows weight distributions of the invented method and WNN method. Fig. 3b shows mean squared errors between multimodal weights of the invented method and WNN and target weights. Target weights 1) for types A and B equal to 1 for modality 1 and 0 for modalities 2 and 3, 2) for types C and D equal to 1 for modality 2 and 0 for modalities 1 and 3, 3) for types E and F equal to 1 for modality 3 and 0 for modalities 1 and 2. The invented method is superior in terms of separation of lineages across all modalities and accuracy of generated joint embedding.

### Applications of the method on multimodal single-cell datasets

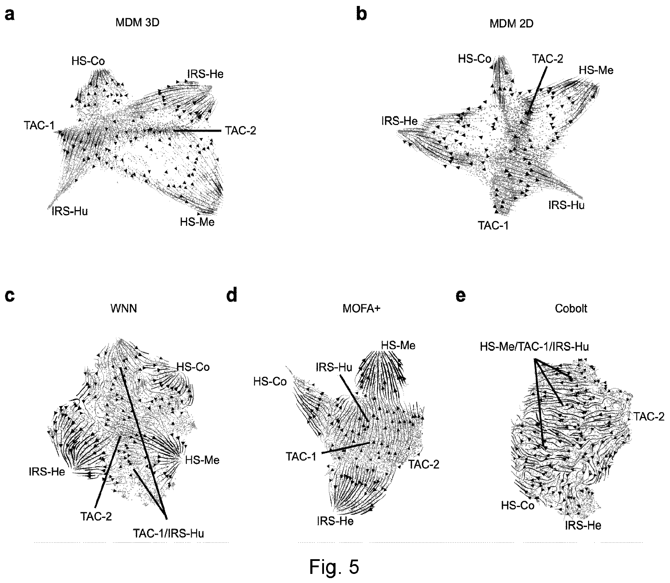

The second presented application of the method is shown in Fig. 4. It is based on the multimodal dataset of hair follicle cells (available at the Gene Expression Omnibus under the accession number GSE140203 - https://www.ncbi.nlm.nih.gov/geo/query/acc.cgi?acc=GSE140203). The dataset consists of two modalities: chromatin accessibility (ATAC-seq) and gene expression (RNA-seq). Fig. 4a shows schematic directions of differentation for major cell types. Fig. 4b shows the annotated visualization of the hair follicle dataset created with the use of the invented method with scVelo RNA velocity transition probabilities between cells. Fig. 4b also visualizes a stream of arrows showing differentiation direction according to RNA velocities. Transient amplifying cells (TACs) are the progenitors of the investigated developmental biological system. The modality weights of the invented method show that the ATAC-seq modality is more informative about TACs (Fig. 4c and 4d) - which is biologically accurate, as chromatin accessibility changes along the developmental trajectory precedes the changes in transcription rates. The invented method was also benchmarked, as said above, against other state-of-the-art methods creating multimodal single-cell embeddings: WNN, MOFA+, and Cobolt. Fig. 4e shows that MDM's computational time when running on the 16CPU machine (using both approximated (ANNs) and exact (ENNs) nearest neighbors) is noticeably faster than other methods. Fig. 4f shows the cohesiveness score, which measures how well visualizations portray ATAC-seq motifs and RNA-seq gene signatures - the higher the score, the better performance. Fig. 4g shows example gene signatures from Yang et al., 2017, which were used for cell annotation [H. Yang et. al, Epithelial-Mesenchymal Micro-niches Govern Stem Cell Lineage Choices, Cell, Volume 169, Issue 3, 20 April 2017, pp. 483-496]. The invented method is capable of reconstructing cell populations, which other methods (WNN, MOFA+, Cobolt) failed to detect. Fig. 5 shows 2D embeddings of the invented method and WNN, MOFA+, Cobolt. Only the invented method captures clearly the cell population of the Huxley's layer of the inner root sheath (IRS-Hu). Fig. 5a shows a MDM embedding visualized in 3D and then projected onto 2D. Fig. 5b shows the MDM embedding visualized in 2D. Fig. 5c shows the WNN embedding visualized in 2D. Fig. 5d shows the MOFA+ embedding visualized in 2D. Fig. 5e shows the Cobolt embedding visualized in 2D. To sum up, the invented method is of higher sensitivity of capturing cell states and determining the cell fate decisions as compared to state-of-the-art methods.

The third presented application of the method shown in Fig. 6. It is based on the dataset of human bone marrow cells (available at the Gene Expression Omnibus under accession number GSE156478 - https://www.ncbi.nlm.nih.gov/geo/query/acc.cgi?acc=GSE156478). The dataset consists of chromatin accessibility (ATAC-seq) and protein epitopes. The annotated visualization of the human bone marrow dataset using the invented method is presented in Fig. 6a, Fig. 6b and 6c show that MDM learned that chromatin accessibility is more informative about early-stage differentiation cells such as hematopoietic stem cells (HSCs). In contrast, differentiated cells such as monocytes, T-cells, or B-cells have higher protein weights. It agrees with the central dogma of molecular biology, which in a simplistic version states that *DNA makes RNA, and RNA makes protein.* To sum up, the invented method is general and determines with high accuracy the cell states and their fates.

The used set-up, including all parameters of the scan, as well as software and hardware used, are exemplary and should not be treated as limiting the scope of the present invention.

**Claims**

1. A method of visualization of developmental landscapes from single-cell multimodal data, comprising the following steps:

   a) Providing input data for two or more modalities, relating to cells being subject to investigations, each modality **characterized by** a set of investigated features, the data for each modality being in a form of a matrix presenting quantitative intensity of each of the investigated features in each of the cells being subject to investigations;
   b) Pre-processing said input data acquired in step a) for each of said two or more modalities so as to reduce the noise level of said input data;
   c1) Processing the pre-processed input data obtained in step b) by modelling separately each of said two or more modalities as a diffusion process with an adaptive Gaussian kernel, so as to obtain an unimodal Markov chain matrix for each of said two or more modalities;
   c2) Calculating multimodal weights for each of the cells being subject to investigations and for each of said two or more modalities;
   d) Calculating a multimodal Markov chain matrix, wherein consecutive values in each particular row of values in the multimodal Markov chain matrix, corresponding to a particular cell being subject to investigations, are calculated by adding corresponding values from the corresponding row of each of the unimodal Markov chain matrix obtained for each of said two or more modalities in step c1) weighted with the corresponding multimodal weights obtained for said cell and the modality in question in step c2);
   e) Eigendecomposing the multimodal Markov chain matrix obtained in step d) so as to obtain a diffusion-based dimensionally reduced Multimodal Diffusion Maps, (MDM,) representation of the two or more modalities;
   f) Visualizing a developmental landscape based on the joint Multimodal Diffusion Maps, MDM, representation obtained in step e), reduced to two dimensions or three dimensions with inclusion of cell fate probabilities.

2. The method according to claim 1, wherein providing the input data in step a) consists in measuring said data in an experiment.

3. The method according to claim 1 or 2, wherein said two or more modalities are selected from: transcriptome, proteome or epigenome.

4. The method according to claim 1, 2 or 3, wherein the pre-processing in step b) is conducted by topic modelling with Bayesian generative methods.

5. The method according to claim 1, 2, 3 or 4, wherein calculating multimodal weights in step c2) is conducted by comparing distances among investigated cells' nearest neighbors from investigated two or more modalities using modality-wise empirical global distributions of distances between cells.

6. The method according to any one of claims from 1 to 5, wherein the eigendecomposition in step e) is conducted by applying ARPACK Software.

7. The method according to any one of claims from 1 to 6, wherein the visualisation in step f) is conducted by reducing the MDM representation to 2 or 3 dimensions with any dimension reduction algorithm or reducing the MDM representation to 2 or 3 dimensions with a force-directed layout ForceAtlas2 algorithm, including transition probabilities between cells such as Velocyto, scVelo or Waddington Optimal Transport.

Fig. 1

**a** Trajectories

Developmental direction

A  B

C  D

E  F

**b** Simulated data

Modality 1 (M1)

A  B  C/D  E/F

Modality 2 (M2)

A/B  C  D  E/F

Modality 3 (M3)

A/B/C  D  E  F

**c** Inferred weights

Modality weight  1  0

M1  M2  M3

Modality weight

M1  M2  M3

A  B  C  D  E  F

**d** MDM-inferred trajectories

C  B  D  A  F  E

Fig. 2

Fig. 3

Fig. 4

Fig. 5

Fig. 6

| | Europäisches Patentamt<br>European Patent Office<br>Office européen des brevets | **EUROPEAN SEARCH REPORT** | **Application Number**<br>EP 23 16 9340 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAY S STANLEY III ET AL: "Manifold Alignment with Feature Correspondence", ARXIV.ORG, CORNELL UNIVERSITY LIBRARY, 201 OLIN LIBRARY CORNELL UNIVERSITY ITHACA, NY 14853,<br>30 September 2018 (2018-09-30), XP081368318,<br>* Abstract; page 3, "Diffusion Maps"; page 5, "Bandlimited correlation"; page 8, "Multimodal Data Fusion" * | 1-7 | INV.<br>G16B45/00 |
| X | JAIN MIKA SARKIN ET AL: "MultiMAP: dimensionality reduction and integration of multimodal data",<br>GENOME BIOLOGY,<br>vol. 22, no. 1,<br>1 December 2021 (2021-12-01), XP055979194,<br>DOI: 10.1186/s13059-021-02565-y<br>Retrieved from the Internet:<br>URL:https://genomebiology.biomedcentral.com/counter/pdf/10.1186/s13059-021-02565-y.pdf><br>* page 12, 3rd full para.; figure 3 * | 1-7 | |
| X | LI BO ET AL: "Cumulus provides cloud-based data analysis for large-scale single-cell and single-nucleus RNA-seq",<br>NATURE METHODS, NATURE PUBLISHING GROUP US, NEW YORK,<br>vol. 17, no. 8, 27 July 2020 (2020-07-27), pages 793-798, XP037208344,<br>ISSN: 1548-7091, DOI: 10.1038/S41592-020-0905-X<br>[retrieved on 2020-07-27]<br>* Abstract; figure 1 * | 1-7 | **TECHNICAL FIELDS SEARCHED (IPC)**<br><br>G16B |

-/--

The present search report has been drawn up for all claims

| Place of search | Date of completion of the search | Examiner |
|---|---|---|
| Munich | 7 August 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number

EP 23 16 9340

## DOCUMENTS CONSIDERED TO BE RELEVANT

| Category | Citation of document with indication, where appropriate, of relevant passages | Relevant to claim |
|---|---|---|
| X | HAO YUHAN ET AL: "Integrated analysis of multimodal single-cell data", CELL, ELSEVIER, AMSTERDAM NL, vol. 184, no. 13, 31 May 2021 (2021-05-31) , page 3573, XP086676067, ISSN: 0092-8674, DOI: 10.1016/J.CELL.2021.04.048 [retrieved on 2021-05-31] * the whole document * ----- | 1-7 |

CLASSIFICATION OF THE APPLICATION (IPC)

TECHNICAL FIELDS SEARCHED (IPC)

The present search report has been drawn up for all claims

| Place of search | Date of completion of the search | Examiner |
|---|---|---|
| Munich | 7 August 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JUNYUE CAO et al.** Joint profiling of chromatin accessibility and gene expression in thousands of single cells. *Science,* 2018, vol. 361 (6409), 1380-1385
- **CHENXU ZHU et al.** An ultra high-throughput method for single-cell joint analysis of open chromatin and transcriptome. *Nature structural & molecular biology,* 2019, vol. 26 (11), 1063-1070
- **SONG CHEN ; BLUE B LAKE ; KUN ZHANG.** High-throughput sequencing of the transcriptome and chromatin accessibility in the same cell. *Nature biotechnology,* 2019, vol. 37 (12), 1452-1457
- **SAI MA et al.** Chromatin potential identified by shared single-cell profiling of RNA and chromatin. *Cell,* 2020, vol. 183 (4), 1103-1116
- **BOYING GONG ; YUN ZHOU ; ELIZABETH PURDOM.** Cobolt: Joint analysis of multimodal single-cell sequencing data. *bioRxiv,* 2021
- **GAOYANG LI et al.** A deep generative model for multi-view profiling of single-cell RNA-seq and ATAC-seq data. *Genome biology,* 2022, vol. 23 (1), 1-23
- **YUHAN HAO et al.** Integrated analysis of multimodal single-cell data. *Cell,* 2021, vol. 184 (13), 3573-3587
- **RICARD ARGELAGUET et al.** MOFA+: a statistical framework for comprehensive integration of multi-modal single-cell data. *Genome biology,* 2020, vol. 21 (1), 1-17
- **HAGHVERDI LALEH.** Diffusion maps for high-dimensional single-cell analysis of differentiation data. *Bioinformatics,* September 2015, vol. 31 (18), 2989-2998
- **LINDENBAUM OFIR.** Multi-view diffusion maps. *Information Fusion,* 2020, vol. 55, 127-149
- **COLE TRAPNELL et al.** The dynamics and regulators of cell fate decisions are revealed by pseudotemporal ordering of single cells. *Nature biotechnology,* 2014, vol. 32 (4), 381-386
- **GIOELE LA MANNO et al.** RNA velocity of single cells. *Nature,* 2018, vol. 560 (7719), 494-498
- **VOLKER BERGEN et al.** Generalizing RNA velocity to transient cell states through dynamical modeling. *Nature biotechnology,* 2020, vol. 38 (12), 1408-1414

- **GEOFFREY SCHIEBINGER et al.** Optimal-transport analysis of single-cell gene expression identifies developmental trajectories in reprogramming. *Cell,* 2019, vol. 176 (4), 928-943
- **RONALD R COIFMAN ; STÉPHANE LAFON.** Diffusion maps. *Applied and computational harmonic analysis,* 2006, vol. 21 (1), 5-3
- **LYLA ATTA ; ARPAN SAHOO ; JEAN FAN.** VeloViz: RNA velocity-informed embeddings for visualizing cellular trajectories. *Bioinformatics,* 2022, vol. 38 (2), 391-396
- **BLEI DAVID M. et al.** Latent Dirichlet Allocation. *Journal of Machine Learning Research,* 2003, vol. 3, 993-1022
- **COIFMAN RONALD R. ; LAFON STÉPHANE.** Diffusion Maps. *Applied and Computational Harmonic Analysis,* July 2006, vol. 21 (1), 5-30
- **LA MANNO G. et al.** RNA velocity of single cells. *Nature,* 2018, vol. 560, 494-498
- **BERGEN V.** Generalizing RNA velocity to transient cell states through dynamical modeling. *Nature Biotechnology,* 2020, vol. 38, 1408-1414
- **SCHIEBINGER G. et al.** Optimal-Transport Analysis of Single-Cell Gene Expression Identifies Developmental Trajectories in Reprogramming. *Cell,* 07 February 2019, vol. 176 (4), 928-943
- **RONALD R COIFMAN ; STÉPHANE LAFON.** Diffusion maps. *Applied and computational harmonic analysis,* 2006, vol. 21 (1), 5-30
- **F. PEDREGOSA et al.** Scikit-learn: Machine Learning in Python. *Journal of Machine Learning Research,* 2011, vol. 12, 2825-2830
- **RICHARD B LEHOUCQ ; DANNY C SORENSEN ; CHAO YANG.** ARPACK users' guide: solution of large-scale eigenvalue problems with implicitly restarted Arnoldi methods. *SIAM,* 1998
- **ALEH HAGHVERDI ; FLORIAN BUETTNER ; FABIAN J THEIS.** Diffusion maps for high-dimensional single-cell analysis of differentiation data. *Bioinformatics,* 2015, vol. 31 (18), 2989-2998
- **LELAND MCLNNES ; JOHN HEALY ; JAMES MELVILLE.** Umap: Uniform manifold approximation and projection for dimension reduction. *arXiv preprint arXiv:1802.03426,* 2018
- **MATHIEU JACOMY et al.** ForceAtlas2, a continuous graph layout algorithm for handy network visualization designed for the Gephi software. *PloS one,* 2014, vol. 9 (6), e98679

- **VOLKER BERGEN et al.** Generalizing RNA velocity to transient cell states through dynamical modelling. *Nature biotechnology,* 2020, vol. 38 (12), 1408-1414
- **SAMUEL L WOLOCK ; ROMAIN LOPEZ ; ALLON M KLEIN.** Scrublet: computational identification of cell doublets in single-cell transcriptomic data. *Cell systems,* 2019, vol. 8 (4), 281-291
- **TIM STUART et al.** Single-cell chromatin state analysis with Signac. *Nature Methods,* 2021
- **ELENI P MIMITOU et al.** Scalable, multimodal profiling of chromatin accessibility, gene expression and protein levels in single cells. *Nature biotechnology,* 2021, vol. 39 (10), 1246-1258
- **BOYING GONG ; YUN ZHOU ; ELIZABETH PURDOM.** Cobolt: integrative analysis of multimodal single-cell sequencing data. *Genome biology,* 2021, vol. 22 (1), 1-21

- Engineering Efficient and Effective Non-metric Space Library. **LEONID BOYTSOV ; BILEGSAIKHAN NAIDAN.** Similarity Search and Applications - 6th International Conference, SISAP. Springer, 02 October 2013, vol. 8199, 280-293
- **MARTIN ESTER et al.** A density-based algorithm for discovering clusters in large spatial databases with noise. *kdd,* 1996, vol. 96 (34), 226-231
- **ALICIA N SCHEP et al.** chromVAR: inferring transcription-factor-associated accessibility from single-cell epigenomic data. *Nature methods,* 2017, vol. 14 (10), 975-978
- **FRIEDMAN, JEROME ; TREVOR HASTIE ; ROBERT TIBSHIRANI.** Sparse inverse covariance estimation with the graphical lasso. *Biostatistics,* 2008, vol. 9 (3), 432-441
- **H. YANG.** Epithelial-Mesenchymal Micro-niches Govern Stem Cell Lineage Choices. *Cell,* 20 April 2017, vol. 169 (3), 483-496